(19) Europäisches Patentamt / European Patent Office / Office européen des brevets

(11) **EP 4 117 514 B1**

(12) **EUROPEAN PATENT SPECIFICATION**

(45) Date of publication and mention
of the grant of the patent:
**30.07.2025   Bulletin 2025/31**

(51) International Patent Classification (IPC):
**A61B 5/02** *(2006.01)*    **A61B 5/0215** *(2006.01)*
**A61B 5/318** *(2021.01)*    **A61B 5/364** *(2021.01)*
**A61B 5/00** *(2006.01)*    **G16H 50/30** *(2018.01)*

(21) Application number: **21708992.9**

(22) Date of filing: **01.03.2021**

(52) Cooperative Patent Classification (CPC):
**A61B 5/02028; A61B 5/0215; A61B 5/318;
A61B 5/364; A61B 5/7267; G16H 50/30;
G16H 50/70**

(86) International application number:
**PCT/EP2021/055014**

(87) International publication number:
**WO 2021/180500 (16.09.2021 Gazette 2021/37)**

(54) **WAVEFORM-BASED HEMODYNAMIC INSTABILITY WARNING**

WELLENFORMBASIERTE HÄMODYNAMISCHE INSTABILITÄTSWARNUNG

AVERTISSEMENT D'INSTABILITÉ HÉMODYNAMIQUE SUR LA BASE D'UNE FORME D'ONDE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **10.03.2020   US 202062987602 P**

(43) Date of publication of application:
**18.01.2023   Bulletin 2023/03**

(73) Proprietor: **Koninklijke Philips N.V.
5656 AG Eindhoven (NL)**

(72) Inventors:
• **NATARAJAN, Annamalai
  5656 AE Eindhoven (NL)**
• **MARIANI, Sara
  5656 AE Eindhoven (NL)**
• **ZONG, Wei
  5656 AE Eindhoven (NL)**

(74) Representative: **Philips Intellectual Property &
Standards
High Tech Campus 52
5656 AG Eindhoven (NL)**

(56) References cited:
**EP-A1- 2 257 217        WO-A1-2015/200750
US-A1- 2018 046 942**

• **HANQING CAO ET AL: "Predicting ICU
hemodynamic instability using continuous
multiparameter trends", ENGINEERING IN
MEDICINE AND BIOLOGY SOCIETY, 2008. EMBS
2008. 30TH ANNUAL INTERNATIONAL
CONFERENCE OF THE IEEE, IEEE,
PISCATAWAY, NJ, USA, 20 August 2008
(2008-08-20), pages 3803 - 3806, XP031508837,
ISBN: 978-1-4244-1814-5**

## EP 4 117 514 B1

**Description**

**BACKGROUND**

Hemodynamic instability (HI) is a condition that may be defined as failure of blood passage to organs and tissues, resulting in an inability to meet metabolic demand. Hemodynamic instability reflects a problem in the circulatory system and can lead to cellular dysfunction and death. Critically ill patients with impaired cardiac performance are at high risk for circulatory failure and hemodynamic instability. In critical care settings, such as the intensive care unit (ICU), intervention to patients with hemodynamic instability often involves fluid resuscitation to increase preload, administration of vasopressors to increase peripheral resistance (afterload) to maintain systemic blood pressure, and/or administration of inotropes to increase contractility of the heart.

Prior works on predicting hemodynamic instability are typically based on nurse-charted vital signs (e.g., body temperature, pulse rate, respiration rate and/or blood pressure) and clinical measurements used to classify time segments in an ICU stay as either stable or unstable. In one study for predicting hemodynamic instability, nurse-charted vital signs and clinical measurements were applied to a model and resulted in an area under the receiver operating characteristics (AUROC) of 0.82. In another study for predicting hemodynamic instability, a result of an AUROC of 0.87 was achieved by excluding missing data from consideration. Another study to predict hemorrhage in a surgical ICU added bedside monitor vital signs, and resulted in an AUROC of 0.92. The bedside monitor vital signs are considered higher frequency than nurse-charted vital signs. Still another study for predicting hemodynamic instability used ECG waveforms, but only on simulated settings on healthy volunteers, and this resulted in an AUROC of 0.86 to 0.88.

Continuous arterial blood pressure (ABP) waveforms carry relevant hemodynamic information. Arterial blood pressure has been used in ICU settings to monitor cardiovascular insufficiency and response to various interventions. The two main components of an individual ABP pulse cycle are the systolic phase and the diastolic phase separated by the dicrotic notch. The closure of the aortic valve is reflected graphically as the dicrotic notch in the ABP pulse cycle. Informative features, such as cardiac output (CO) as a measure of cardiac performance, can be estimated from ABP waveforms. Cardiac output is a function of heart rate, preload, contractility, and afterload. Other metrics such as the maximum of the first derivative of an ABP pulse cycle serve as an indirect measure of the contractility of the heart. ABP waveforms have been used to predict hypotensive episodes in ICU settings. For example, in one study to predict a hypotensive event in the next fifteen minutes, features extracted from ABP waveforms resulted in an AUROC in the range of 0.91 to 0.97.

WO 2015/200750 A1 discloses a method for developing models for classification of physical conditions of a subject based on monitored physiological signal data.

Reference is made to the paper: HANQING CAO ET AL: "Predicting ICU hemodynamic instability using continuous multi parameter trends", ENGINEERING IN MEDICINE AND BIOLOGY SOCIETY, 2008. 30TH ANNUAL INTERNATIONAL CONFERENCE OF THE IEEE, 20 August 2008. This discloses methods for predicting ICU hemodynamic instability using continuous physiological parameter data.

**SUMMARY**

According to an aspect of the present disclosure, an apparatus includes a first interface, a memory and a processor. The first interface interfaces at least one electrocardiogram monitor monitoring a patient. The memory stores instructions. The processor executes the instructions. When executed by the processor, the instructions cause the apparatus to identify a plurality of heart beats from electrocardiogram waves received via the first interface from the at least one electrocardiogram monitor; separate the plurality of heart beats into first temporal windows, and extract features of the heart beats in each of the first temporal windows as first extracted features for each first temporal window. The instructions also cause the apparatus to generate, based on the first extracted features, generated features across a second temporal window that includes a plurality of the first temporal windows; apply trained artificial intelligence to the generated features; predict hemodynamic instability for the patient based on applying the trained artificial intelligence to the generated features, and output an alert warning of the hemodynamic instability based on predicting the hemodynamic instability.

According to another aspect of the present disclosure, a method includes receiving, via a first interface that interfaces at least one electrocardiogram monitor monitoring a patient, a plurality of electrocardiogram waves; identifying a plurality of heart beats from the electrocardiogram waves; separating the plurality of heart beats into first temporal windows, and extracting features of the heart beats in each of the first temporal windows as first extracted features for each first temporal window. The method also includes generating, based on the first extracted features, generated features across a second temporal window that includes a plurality of the first temporal windows; applying trained artificial intelligence to the generated features; predicting hemodynamic instability for the patient based on applying the trained artificial intelligence to the generated features, and outputting an alert warning of the hemodynamic instability based on predicting the hemodynamic instability.

According to yet another aspect of the present disclosure, a tangible non-transitory computer readable storage medium stores a computer program. When executed by a processor, the computer program causes a system that includes the tangible non-transitory computer readable storage medium to identify a plurality of heart beats from electrocardiogram waves received via a first interface from at least one electrocardiogram monitor; separate the plurality of heart beats into first temporal windows, and extract features of the heart beats in each of the first temporal windows as first extracted features for each first temporal window. The computer program also causes the system to generate, based on the first extracted features, generated features across a second temporal window that includes a plurality of the first temporal windows; apply trained artificial intelligence to the generated features; predict hemodynamic instability for the patient based on applying the trained artificial intelligence to the generated features, and output an alert warning of the hemodynamic instability based on predicting the hemodynamic instability.

## BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.

FIG. 1 illustrates a system for waveform-based hemodynamic instability warning, in accordance with a representative embodiment.
FIG. 2 illustrates processing pipelines and extracted features for waveform-based hemodynamic instability warning, in accordance with a representative embodiment.
FIG. 3 illustrates a method for waveform-based hemodynamic instability warning, in accordance with a representative embodiment.
FIG. 4 illustrates a computer system for waveform-based hemodynamic instability warning, in accordance with a representative embodiment.

## DETAILED DESCRIPTION

In the following detailed description, for the purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms 'a', 'an' and 'the' are intended to include both singular and plural forms, unless the context clearly dictates otherwise. Additionally, the terms "comprises", and/or "comprising," and/or similar terms when used in this specification, specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to", "coupled to", or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments. Such methods and apparatuses are within the scope of the present disclosure.

As described herein, ECG waveforms and/or ABP waveforms may be used to predict hemodynamic instability, both without and in combination with nurse-charted vital signs and clinical measurements. According to embodiments herein, if a baseline model for hemodynamic instability predictions is established using only clinical measurements and nurse-charted vital signs, an improvement of 5% in an AUROC may be obtained when features extracted from ECG waveforms and ABP waveforms are added, along with a 9% improvement in area under precision recall curve (AUPRC). When using the features extracted from ECG waveforms and ABP waveforms in isolation without any clinical measurements or nurse-charted vital signs, a 4% improvement in AUROC and AUPRC may still be observed when compared to the baseline results.

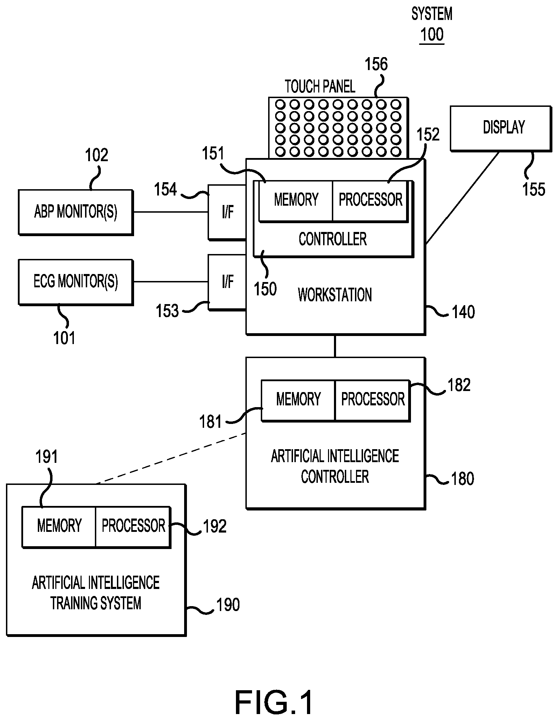

FIG. 1A illustrates a system 100 for waveform-based hemodynamic instability warning, in accordance with a representative embodiment.

The system 100 in FIG. 1A is a system for waveform-based hemodynamic instability warning and includes components that may be provided together or that may be distributed. The system 100 includes one or more ECG monitor(s) 101, one or more ABP monitor(s) 102, a workstation 140, a monitor 155, and an artificial intelligence controller 180. An artificial intelligence training system 190 provides trained artificial intelligence to the artificial intelligence controller 180.

The ECG monitor(s) 101 are electrocardiogram monitors. An ECG monitor monitors electrical activity of a heart by taking measurement of the electrical current that runs through the heart. Each ECG monitor may record the strength and timing of signals in a graph, and may include one or more electrodes attached to the body of a patient via patches and a wire that carries the ECG trace signal to a receiver on the workstation 140. The signals recorded by the ECG monitor(s) 101 may include P-waves, QRS complexes, and T-waves.

The ABP monitor(s) 102 are arterial blood pressure monitors. The ABP monitor(s) 102 monitor arterial blood pressure of a patient via a pressure transducer. The pressure transducer may be an external pressure sensor connecting to an arterial vessel. The external pressure sensor typically connects to the arterial vessel at the radial location via a fluid-filled catheter that enters into the arterial vessel. The pressure transducer may alternatively be an internal pressure sensor that is mounted at the tip of a catheter that enters into the arterial vessel. Continuous ABP signal waveforms may be recorded and arterial blood pressure measurements may be measured by the ABP monitor(s) 102. Arterial blood pressure measurements that may be measured using the ABP monitor(s) 102 include systolic blood pressure (SPB), diastolic blood pressure (SBP) and mean arterial pressure (MAP). The ABP monitor(s) 102 connect to a receiver on the workstation 140.

The workstation 140 includes a controller 150, a first interface 153, a second interface 154, and a touch panel 156. The controller 150 includes a memory 151 that stores instructions and a processor 152 that executes the instructions. The controller 150 controls and implements some or all aspects of methods attributable to the workstation 140 as described herein. The first interface 153 interfaces the ECG monitor(s) 101 to the workstation 140. The second interface 154 interfaces the ABP monitor(s) 102 to the workstation 140. That is, the second interface 154 interfaces one or more arterial blood pressure monitor. The first interface 153 and the second interface 154 may be ports, adapters, or other types of wired or wireless interfaces used to transmit and receive data. The touch panel 156 may include a touch interface that accepts input via touch such as through direct touch or via a mouse, keyboard or other hand-controlled input mechanism. The touch panel 156 may also include a visual display for displaying touch input so that a user can confirm the input. The workstation 140 may also include one or more other input interface(s). The other input interface(s) (not shown) of the workstation 140 may include ports, disk drives, wireless antennas, or other types of receiver circuitry. The other input interface(s) may further connect other user interfaces, such as a mouse, a keyboard, a microphone, a video camera, a touchscreen display, or another element or component to the workstation 140.

The monitor 155 is an electronic monitor that displays visualizations of images and data. The monitor 155 may be a computer monitor, a display on a mobile device, a television, an electronic whiteboard, or another screen configured to display electronic imagery. The monitor 155 may also include one or more input interface(s) such as those noted above that may connect other elements or components to the workstation 140, as well as a touch screen that enables direct input via touch.

The artificial intelligence controller 180 includes a memory 181 that stores instructions and a processor 182 that executes the instructions. The artificial intelligence controller 180 may dynamically apply trained artificial intelligence to data received from the controller 150 based on ECG signals from the ECG monitor(s) 101 and based on ABP signals from the ABP monitor(s). 102. In some embodiments, the artificial intelligence controller 180 is implemented as a component of the workstation 140. In alternative embodiments, the controller 150 and the artificial intelligence controller 180 are implemented by the same, rather than different, memories and processors.

The artificial intelligence training system 190 includes a memory 191 that stores instructions and a processor 192 that executes the instructions. The artificial intelligence training system 190 may train artificial intelligence based on data sets from numerous different instantiations of patient monitoring so as to learn optimal correlations between input data and a resulting outcome of hemodynamic instability. The data sets may be provided from a database such as the Medical Information Mart for Intensive Care (MIMIC) III database described elsewhere herein. As a result, trained artificial intelligence provided from the artificial intelligence training system 190 may be used to predict hemodynamic instability in the manner described herein, and ultimately to provide alerts so as to result in treatments to avoid, treat or otherwise address the predicted hemodynamic instability. The training may be based on analysis of thousands of patients including hundreds of hemodynamically unstable patients. The training may involve binary classification, and may result in a 5% or better improvement in an AUROC when features from both ECG waveforms and ABP waveforms are considered along with laboratory results and nurse-charted vital signs. The training may also result in a 4% or better improvement in an AUROC when only features from both ECG waveforms and ABP waveforms are considered.

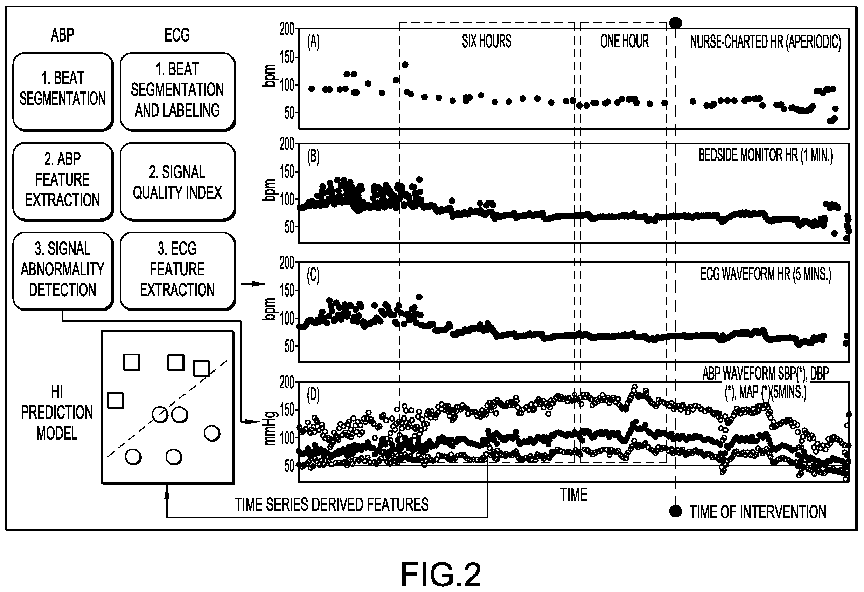

FIG. 2 illustrates processing pipelines and extracted features for waveform-based hemodynamic instability warning, in accordance with a representative embodiment.

In FIG. 2, an ABP processing pipeline in the upper left is end-to-end and includes three steps of (1) beat segmentation, (2) ABP feature extraction and (3) signal abnormality detection. The end-to-end ABP processing pipeline is used for feature extraction from ABP waveforms. An ECG pipeline in the upper left is also end-to-end and also includes three steps of (1) beat segmentation and labeling, (2) signal quality index and (3) ECG feature extraction. The beat segmentation in the ECG pipeline may include detection of a QRS complex and then classification of the beat. The signal quality index in the ECG pipeline may include a signal quality assessment. The ECG waveforms in the ECG pipeline may be from the MIMIC III database and may be recorded at 125Hz or 250 Hz. The ECG waveforms may be up sampled from 125Hz to 250Hz to increase the time resolution and detectability of the QRS complex. The end-to-end ECG and ABP processing pipelines in FIG. 2 are objective measures which use waveform signals commonly available in ICU settings without requiring any additional special devices.

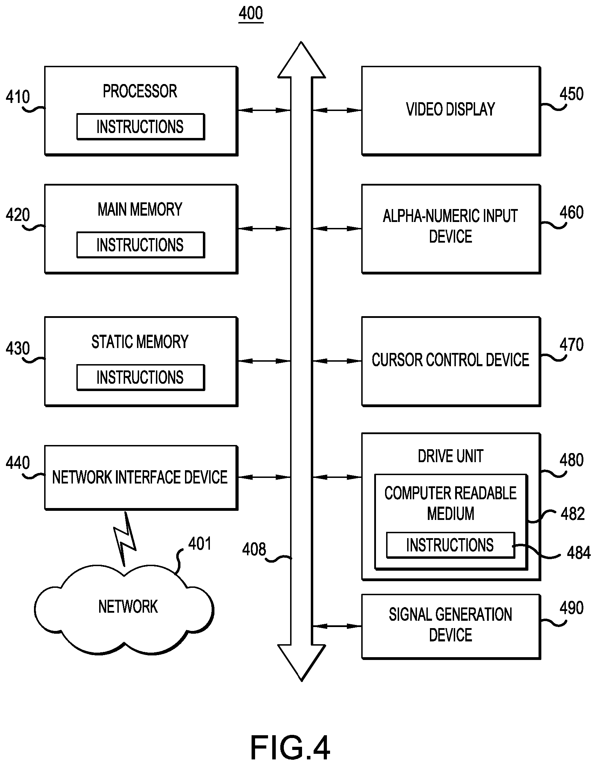

In FIG. 2, panel (A) on the right side in FIG. 2 shows nurse-charted vital signs, and panel (B) on the right side shows bedside monitor vital signs. Data from the ECG pipeline is shown in (C) ECG Waveform HR (5 MINS) on the right side, which is the 5-minute averaged heart rate (HR) extracted from the ECG waveform. Data from the ABP pipeline is shown in (D) ABP Waveform SBP(*), DBP(*), MAP(*) (5 MINS) on the right side, which are the 5-minute averaged systolic blood pressure (SBP), the 5-minute averaged diastolic blood pressure (DBP), and the 5-minute averaged mean arterial pressure (MAP) extracted from ABP waveform, respectively. In FIG. 2, the features in (A), (B), (C) and (D) in FIG. 4 are for a sample patient. The features shown in (C) for the ECG waveform are drawn from heartbeats separated into first temporal windows of five minutes. The features shown in (D) are drawn from arterial blood pressure waves segmented into pulse cycles that are then also separated into the first temporal windows of five minutes.

A six hour window in FIG. 2 is a second temporal window superimposed over (A), (B), (C) and (D). The six hour window ends at least one hour before the time of intervention as shown by a vertical line superimposed over (A), (B), (C) and (D). The six hour window includes seventy two of the first temporal windows which are each five minutes for the ECG Waveforms in (C) and for the ABP Waveforms in (D). Derived features from the six hour window in (A), (B), (C) and (D) are used in a hemodynamic instability prediction model on the lower left to classify stable patients and unstable patients. The derived features are derived from the time series data of (A), (B), (C) and (D) and thus include derived features from the nurse-charted vital signs from (A), derived features from the bedside monitor vital signs from (B), derived features from the segmented ECG Waveforms in (C) and derived features from the ABP Waveforms in D.

The vital signs represented in the nurse-charted vital signs shown in (A) and the bedside monitor vital signs represented in (B) in FIG. 2 include heart rate (HR), systolic blood pressure (SBP) and diastolic blood pressure (DBP). Clinical measurements include numerous features extracted from laboratory test results and ventilator readings, including arterial base excess, aspartate aminotransferase (AST), bands, basophils, calcium, $CO_2$, creatinine, Eosinophils (EOS), fraction of inspired oxygen (FIO2), glucose, hematocrit, hemoglobin, ionized calcium, lactate, magnesium, mean airway pressure, partial pressure of carbon dioxide ($PaCO_2$), peak inspiration pressure, potassium, partial thromboplastin time (PTT), oxygen saturation (Sao2), sodium, bilirubin, white blood cell count, blood urea nitrogen (BUN) and central venous pressure. Clinical measurements may also include body temperature as well as age of the patient at the time of admission.

Clinical measurements may also include mean arterial blood pressure (MAP) computed as $\frac{SBP+2\times DBP}{3}$ and shock index (SI) computed as $\frac{HR}{SBP}$.

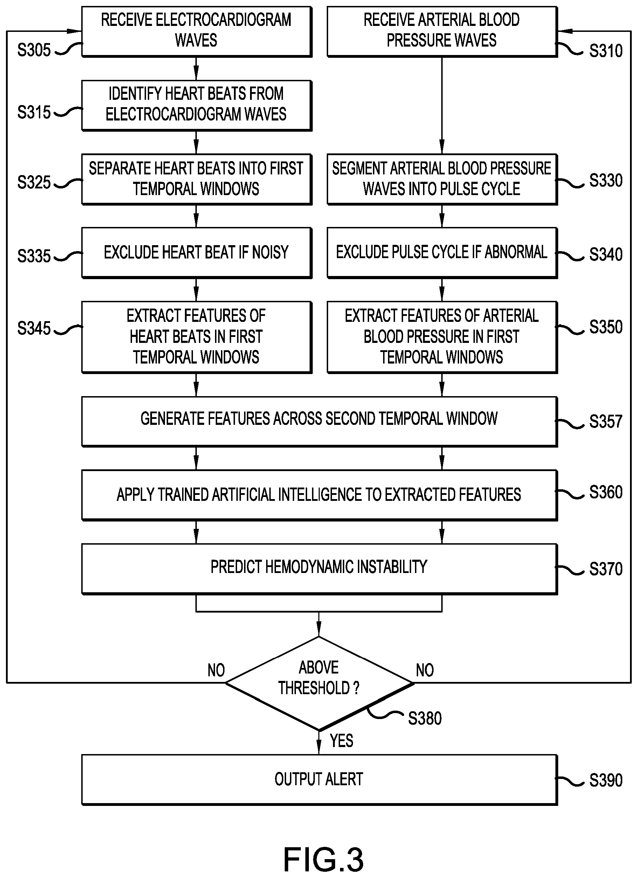

FIG. 3 illustrates a method for waveform-based hemodynamic instability warning, in accordance with a representative embodiment.

FIG. 3 illustrates a waveform-based hemodynamic instability warning process, in accordance with a representative embodiment. The method of FIG. 3 may be performed by a single apparatus such as the controller 150 or the workstation 140, a single system such as the system 100, by or on behalf of a single entity, or by distributed apparatuses, distributed systems, or by or on behalf of multiple entities. In some embodiments, the method of FIG. 3 may be performed by the workstation 140 of FIG. 1 when the workstation 140 is integrated with or includes the artificial intelligence controller 180, or a combination of the workstation 140 and the artificial intelligence controller 180 of FIG. 1 if the workstation 140 is provided separate from the artificial intelligence controller 180.

At S305, the method of FIG. 3 includes receiving electrocardiogram waves. The electrocardiogram waves may be received by the workstation 140 from the ECG monitor(s) 101 via the first interface 153 in FIG. 1

At S310, the method of FIG. 3 includes receiving arterial blood pressure waves. The arterial blood pressure waves may be received by the workstation 140 from the ABP monitor(s) 102 via the second interface 154 in FIG. 1.

At S315, the method of FIG. 3 includes identifying heart beats from the electrocardiogram waves. The heart beats may be identified from the electrocardiogram waves received at S305, and may be identified from an analysis performed by the controller 150 in FIG. 1.

At S325, the method of FIG. 3 includes separating heart beats into first temporal windows. The heart beats may be separated into first temporal windows by the controller 150 in FIG. 1. For example, the first temporal windows may each include five minute intervals, and as described herein, seventy two of the first temporal windows may be included in each second temporal window of six hours.

At S330, the method of FIG. 3 includes segmenting the arterial blood pressure waves into pulse cycles. For example, the controller 150 in FIG. 1 may segment arterial blood pressure waves into pulse cycles. Contiguous arterial blood pressure waveforms may be segmented into individual pulse cycles using a pulse detection algorithm which detects the arterial blood pressure pulse onset.

Additionally, although not shown, the pulse cycles may be separated into first intervals. For example, the heart beats and the pulse cycles of arterial blood pressure waves may be separated into the same first temporal windows.

At S335 the method of FIG. 3 includes excluding a heartbeat from further processing if the heartbeat is noisy. The exclusion at S335 may be performed by the controller 150 in FIG. 1, and may involve one or more than one heartbeat. Correct extraction of ECG features is contingent on the ECG signal quality, and a number of factors may affect the quality of the ECG signal, including patient movement, poor electrode contact, misplacement of electrodes, and electrical interference. The ECG quality assessment measure may be based on a combination of existing criteria and by accounting for various the types of noise that are present in the data. Eight types of noise that may be accounted for and filtered by a noise filter include low frequency noise, high frequency noise, flat line ratio, peak-to- peak amplitude, spike index with slope, saturation of amplitude, power-line noise, and outlier ratio of detected beat-to-beat (QRS-to-QRS, or RR) intervals. For each type of noise, an empirically determined threshold may be tuned using ECG data from a separate group of patients. The overall signal quality index (SQI) may be computed using the eight sub-indices. The signal quality may be assessed on a second-by-second basis. For a one-second ECG segment, the overall SQI receives a value 1 (acceptable) only if all the sub-indices are below the tuned thresholds.

At S340, the method of FIG. 3 includes excluding an ABP pulse cycle from further processing if the pulse cycle is abnormal noisy. That is, the method of FIG. 3 may include excluding at least one individual pulse cycle identified as abnormal from application of the trained artificial intelligence based on identifying the at least one individual pulse cycle as abnormal. The exclusion at S340 may be performed by the controller 150 in FIG. 1, and may involve one or more than one pulse cycle. Signal abnormality index (SAI) may be computed on individual arterial blood pressure pulse cycles to discard abnormal arterial blood pressure pulse cycles deemed unusable. An ABP pulse cycle may be discarded when any one of the following conditions are met: (1) SBP > 300 mmHg or DBP < 20 mmHg; (2) MAP < 30 mmHg or > 200 mmHg; (3) (pulse pressure) PP is < 20 mmHg; (4) Sum of negative slope of the ABP pulse cycle < -40 mmHg / 100 milliseconds; (5) Difference between SBP and DBP between adjacent ABP cycles exceed 20 mmHg; (6) Difference between length of ABP pulse cycles in seconds exceed $\frac{2}{3}$ 's of a second; (7) Inconsistent ordering of SBP, dicrotic notch and DBP; (8) SBP < DBP.

At S345, the method of FIG. 3 includes extracting features of heart beats in the first temporal windows. ECG waveforms may be leveraged by extracting heart rate at higher temporal resolutions and deriving heart rate variability (HRV) to serve as indirect measures of autonomic control and vasomotor tone. Examples of features extracted from heart beats in first temporal windows may include averages computed from individual beats, such as average heart rates. Additionally, examples of features extracted from heart beats in first temporal windows may include features over all beats inside a first temporal window, such as heart rate variability. A commercial-grade (FDA-approved) ECG arrhythmia detection algorithm may be employed to perform ECG beat detection and classification for each lead of ECG in each patient. For each detected beat, the beat position may be assigned to the QRS peak, and the beat type may be assigned to one of the following predetermined labels based on characteristics of each heart beat: Normal beat (N), Ventricular ectopic beat (V), Super-ventricular premature beat (S), Paced beat (P), Questionable/unclassified beat (Q), and Learning beat (L).

The following ECG features may be extracted/computed per ECG lead using the beat position and labels only from the segments with good signal quality. When ECG features are available from multiple leads, features may be chosen from a single lead, such as in the order: II, V, MCL, aVF, III, aVR, aVL, V1, V3, V5 and V2. The ECG leads may be ranked in order based on availability and clinical relevance.

Heart rate (HR) is the number of heart beats per minute. The instantaneous heart rate may be computed using the R-R interval between two adjacent beats with beats labeled as N, V, S, P, Q, or L. The average of the instantaneous HR values may be taken over each first temporal window of five minutes. Time series of HR values may be added from (A) nurse-charted vital signs, (B) bedside monitor vital signs measured every minute and (C) ECG waveforms, averaged over five minute windows.

Another extracted ECG feature may be heart rate asymmetry (HRA). HRA quantifies the rapid acceleration and deceleration of heart rate over a period of time, and succinctly represents the imbalance as a single index.

An additional extracted ECG feature may be heart rate variability (HRV). HRV is the fluctuation in the time intervals between adjacent normal heart beats. HRV metrics over five minute temporal windows are computed using only the beats that are labeled 'N'. HRV metrics are grouped into time, frequency and non-linear domains as explained below. Time domain HRV metrics include the standard deviation of NN intervals (SDNN), the root mean square of successive differences between normal heartbeats (RMSSD), the standard deviation of successive NN interval differences (SDSD), the percent of adjacent NN intervals that differ from one another by more than 20 milliseconds (pNN20) and 50 milliseconds (pNN50) respectively.

Additional extracted ECG features may be frequency domain HRV metrics that leverage fast Fourier transform (FFT) on the NN interval time series to extract the contributions of different frequency components. The frequency components may be organized into four nonoverlapping bands as ultra low frequency (0.0001 Hz to 0.003 Hz), very low frequency (0.004 to 0.04 Hz), low (0.05 to 0.15 Hz) and high frequency (0.16 to 0.4 Hz). These frequency domain metrics may require computation on adjacent NN intervals without any gaps. Hence, these metrics may be computed only on the longest, contiguous segment of NN intervals inside a five minute window. For this segment, the NN intervals may be resampled using cubic interpolation at a sampling frequency of 4Hz to obtain regularly and frequently sampled time series. The mean of NN time series may be subtracted to remove effects of baseline offsets. The Welch periodogram may be programmed on this time series using a Hanning window. NN time series of length $\leq 255$ samples may be discarded since the Hanning window was set to a minimum length of 256 samples. The frequency domain metrics include power in the four frequency bands, total power, normalized and relative power in low (LF) and high frequency (HF) bands and $\frac{LF}{HF}$ ratio. The correlation between heart rate and HRV computed in the six hour second temporal window may also be added to the features.

Several non-linear HRV metrics may also be included. These metrics may be computed on the Poincare plot of the longest segment of adjacent NN intervals, the same as the frequency domain analysis above. An ellipse may be fitted to the points on a Poincare plot and the width (SD1) and length (SD2) of the ellipse may be computed along with the $\frac{SD1}{SD2}$ ratio.

The number of premature beat counts may be tracked by tracking the number of premature ventricular contraction (PVC) beats and super-ventricular premature contraction beats counts within each five minute window. The frequency of PVC is a predictor of heart failure and death via a decrease in left ventricular ejection fraction.

At S350, the method of Fig. 3 includes extracting features of arterial blood pressure in the first temporal windows. ABP waveforms may be leveraged by extracting blood pressure measurements at relatively-high temporal resolutions as well as ABP morphological features. The morphological features may be used to estimate CO, contractility of heart and other indicators of hemodynamic states.

In each ABP pulse cycle, SBP may be computed as the local maximum in the ABP pulse cycle and DBP as the first point with zero slope when traversing the ABP pulse cycle from right to left. DBP may be computed in this manner rather than using the minimum. From the SBP and DBP, MAP and pulse pressure (PP) may be computed. To locate dicrotic notch two approaches may be used: one, as $0.3 \times T$ where T is the length of the ABP pulse cycle in seconds, and two, as the first zero slope crossing following the SBP. Both approaches locate a dicrotic notch to be in places where there is no clear separation between systole and diastole. Hence, the dicrotic notch is located as the first zero slope crossing following t where t is chosen as round (0.3xT). This combination approach results in fewer errors in locating dicrotic notch as assessed by visual evaluation. Additionally, if the SBP, DBP or dicrotic notch is not found, then the ABP pulse cycle may be excluded from computations at 340.

Derived morphological features include time to SBP, time to DBP, and time to dicrotic notch all from pulse onset, areas under systole, diastole, and full ABP pulse cycle, and length of ABP pulse cycle. The maximum slope (first derivative of the ABP pulse cycle), $(dP/dt)_{max}$, is computed as an indirect estimated measure of the contractile forces in the heart that are used in titration of inotropes. To these features two indirect measures of CO are added, as:

$$CO_{Zander} = \frac{PP}{SBP + DBP} \times HR \qquad CO_{Area} = Area_{systolic} \times HR$$

where, PP, SBP and DBP and systolic area are extracted per ABP pulse cycle. Here, the heart rate that is closest in time to the current ABP pulse cycle may be used. The features are computed per ABP pulse cycle and an average may be taken over each first temporal window of five minutes. In (D) in FIG. 2 above, time series of five minute averaged SBP, DBP and MAP are computed from ABP waveforms for the same patient.

At S357, the method of FIG. 3 includes extracting features across a second temporal window. The ECG and ABP waveforms features that are extracted results in time series as shown in FIG. 2 (C) and (D). The second temporal window may include multiple first temporal windows in a time series as shown in FIG. 2, and may include, for example, seventy two first temporal windows for a total of six hours. The features extracted across the second temporal window may be extracted by computing time series features such as mean, standard deviation and slope of features extracted in the first temporal windows that are included in the second temporal window.

Derived features (DF) that may be extracted across the second temporal window may be extracted from the time series of the first temporal windows to capture relevant information pertaining to hemodynamic instability. As shown in FIG. 2 above, only the time series available in the six hour window is summarized at S357, and not data in the one hour window before the intervention since this provides the physicians adequate time to intervene. Mean, standard deviation, median, min and max may be computed for each time series in a six hour window to capture distribution properties of a time series. The slope and intercept of each time series are computed to capture trend information and baseline shifts respectively. In order to make comparisons meaningful across patients, trend features may be computed on mean subtracted times series. Lastly, the slope and approximate entropy may be computed to capture the regularity and fluctuations in the time series. The pattern length, over which to look for similarity is set to a default of 2 for both approximate and sample entropy.

In an embodiment, multiple second temporal windows may be used and multiple second temporal windows may overlap. For example, when the second temporal windows are six hours, a different second temporal window may start every thirty minutes so that the features generated at S337 may be generated every thirty minutes for example.

At S360, the method of FIG. 3 includes applying trained artificial intelligence to extracted features. The trained artificial intelligence may be trained by the artificial intelligence training system 190 and provided to the artificial intelligence controller 180. The trained artificial intelligence may be applied by the artificial intelligence controller 180, or by the workstation 140 when the artificial intelligence controller 180 is provided by the controller 150.

At S370, the method of FIG. 3 includes predicting hemodynamic instability. For example, hemodynamic instability may be predicted in advance by one or more hours. The prediction may include a confidence level, such as 30%, 60% or 80%. The prediction may also specify a specific time or time period in which the hemodynamic instability is predicted to begin or to be detectable.

At S380, the method of FIG. 3 includes determining whether the prediction of hemodynamic instability is above a threshold. If the predicting of hemodynamic instability is not above a threshold (S380 = No), the process of FIG. 3 returns to S305 and S310. For example, a threshold may be 50% or 75%. In some embodiments, multiple thresholds may be used. For example, a first threshold of 25% may be considered a warning threshold for which clinical staff should prepare to take an action in response to the prediction, and a second threshold of 50% may be considered an action threshold for which clinical staff should take an action in response to the prediction. An alert may include a projection in advance of when the patient will enter a phase of hemodynamic instability based on an estimated likelihood passing a predetermined threshold.

If the predicting of hemodynamic instability is above a threshold (S380 = Yes), the method of FIG. 3 includes outputting an alert warning of the hemodynamic instability. Additional actions to take if the prediction is above a threshold may include treatment or beginning preparations for a treatment.

Additionally, the method of FIG. 3 may use trained artificial intelligence. The training of the trained artificial intelligence may be based on large patient populations with relevant data, such as patients with information stored in the MIMIC III database. The MIMIC III database has both electronic health records (EHR) and waveform records for a matched subset of patients. The EHR includes entries from a variety of laboratory test results, medications, fluid intake, as well as nurse-charted vital signs. The waveform records contain multi-parameter physiological signals including ECG and ABP. The MIMIC III database also includes bedside monitor generated vital signs, such as heart rate and systolic blood pressure (SBP) which are available every minute.

The MIMIC III database includes EHR data from 46,520 unique ICU patients. The MIMIC III database has matched physiological waveforms and bedside monitor vital signs for a subset 10,282 patients. The process of training the trained artificial intelligence may involve first identifying hemodynamically unstable patients who received strong or weak interventions pertaining to hemodynamic instability. Strong interventions may include the use of vassopressors such as dobutamine, dopamine, epinephrine, norepinephrine, phenylephrine, vasopressin and isuprel. Weak interventions may include the use of fluid therapy, packed red blood cells and pharmacological agents e.g., lidocaine. In the MIMIC III database, 15,713 patients may be identified with one or more strong interventions and 9,949 patients were identified with one or more weak interventions. Patients with only weak interventions may be discarded from subsequent processing. The remaining 20,858 patients who did not receive any intervention may be identified as stable patients. Filtering may be performed for stable and unstable patients who had waveforms, and this may result in 4,460 stable patients with waveforms and 3,037 unstable patients with waveforms. Selection may also be limited to unstable patients who had either labs, nurse-charted vital signs, bedside monitor vital signs, ECG or ABP in a temporal window preceding the very first strong intervention since time of hospital admission. The choice of the second temporal windows may be 6 hours up to one hour prior to an intervention in some embodiments described herein. For stable patients, the six hour window may be randomly preselected. Stable and unstable patients with similar levels of care (e.g. at least one nurse charted HR and SBP are available inside of the six hour window) may be used as the basis of the training, and in one set of experiments this resulted in identifying 880 unstable patients and 2,501 stable patients.

The problem of predicting hemodynamically stable from unstable patients may be approached as a binary classification problem. The gradient boosted trees (XGBoost) model may be used as a boosting model with an ensemble of decision trees. In each iteration, a decision tree may be added to the existing collection of trees to correct for errors from the previous iteration with the objective of minimizing a global loss function. The implementation of gradient boosted trees available in Python may be used. Hyperparameter tuning may be used on regularization lambda from 10-4 to 1, learning rate in the range of 10-4 to 0.3 and maximum depth of each decision tree from 1 to 5. All other settings may be set to the defaults. A stratified 5-fold nested cross validation may be performed over all patients. Hyperparameter tuning may be performed on four train folds and a test may be performed on the held out fifth fold. The mean AUROC curve and the mean AUPRC curve over five test folds may be reported. The standard error over five folds may also be reported.

The results from two experiments are reported. In Experiment I, performance of adding features at higher temporal resolutions is compared to a baseline model. The baseline model may utilize only the last entry (at the end of the second temporal window) of clinical measurements and nurse-charted vital signs. The last entries in the six hour window after forward filling both the clinical measurements and nurse-charted vital signs may be used, making every effort to minimize the number of missing features per patient. This experiment includes data from all stable and unstable patients. This experiment simulates a real world ICU setting where the hemodynamic instability prediction model leverages what is available in the ICU to improve prediction performance.

The baseline model (using clinical measurements and vital signs) may have an AUROC of 0.89 and an AUPRC of 0.79. The availability of these features is largely caregiver dependent and indirectly represents clinician concern or other biases. To the baseline model, derived features from nurse-charted vital signs (Nurse DF) and bed side monitor vital signs (Monitor DF) may be added. Adding derived features of the vital signs to the baseline model improves the AUROC by 2% while there is more visible change in AUPRC's. P values from performing a paired t-test of AUROC's, from five folds, to the baseline model may be reported. The improvement in performance from including nurse-charted vital signs exhibits more variance when compared to bedside monitor vital signs. Next, features at higher temporal resolutions are added to the baseline model. Derived features from ABP waveforms, ECG waveforms are added, and features from both ABP and ECG waveforms are combined. These high temporal resolution features may include basic vital signs along with other ABP and ECG specific features. When including both ECG and ABP features, a 4% improvement in AUROC and a 7% improvement in AUPRC compared to the baseline model may be obtained.

In Experiment I, the relative contribution of different features groups is unknown since the availability of feature groups across patients is non-uniform. For example, only a third of the patients have ABP waveforms but almost all patients have ECG waveforms. In order to compare the relative performance of different feature groups, experiment II is performed where only patients who had data from all five feature groups in the six hour window are included, and each feature group is evaluated in isolation. This resulted in just 243 unstable patients and 230 stable patients. All other details in Experiment II are similar to Experiment I.

Using last entries from clinical measurements and nurse-charted vital signs results in an AUROC of 0.82 which is a 7% drop compared to Experiment I, which used a much larger number of patients. A big improvement in AUROC is not observed when using nurse-charted vital signs and monitored vital signs. Lastly, when evaluating the waveform features an AUROC of 0.84 for ABP and 0.85 for ECG+ABP may be obtained. While there is a drop in performance when using only ECG features it is not statistically significant (P=0.36). Small improvements may be obtained in performance when using ABP in isolation versus in conjunction with ECG. This suggests that information available to ABP but not to ECG (e.g., cardiac output or contractility), may be leveraged by the prediction model. Additionally, none of the performances are significantly different from the baseline model. The AUPRC's follow a very similar trend but are better than AUROC's since there are more positive (243 unstable patients) when compared to negative examples. The results in this experiment illustrate that even in the absence of clinical measurements and nurse-charted vital signs, objective measures such as vital signs from bedside monitors and features from waveforms carry relevant information pertaining to hemodynamic instability.

As a last analysis, the clinical relevance of these features is probed and the degree to which these waveform features were representative of the underlying systems (i.e., cardiac performance, autonomic tone and vasomotor tone) is examined. The importance of features is examined as ranked by the XGBoost model from Experiment II over all five CV folds. The smaller cohort is chosen to examine feature importance since all patients have all five feature groups and the proportion of stable to unstable patients are roughly equal (230 vs 243). However within each feature group there could be imbalances in the availability of features which influence their importance. To correct for this, the importance of each feature is subtracted from the importance of its missingness pattern. A data matrix is first created by setting a feature to 1 when present and 0 otherwise - which simply codes for missingness patterns. Second, this binary data matrix is used in the prediction model and feature importance of missingness patterns is computed. Following this, corrected feature importance scores are computed ranging from '1.0' (where a feature is ranked high and its missingness pattern have little to no influence) to '0.0' (where the importance of a feature is completely counteracted by the importance of its missingness patterns) to '-1.0' (where a feature is not important but its missingness pattern carries information on stable vs. unstable patients).

Among the features in clinical measurements-vital signs are SBP, MAP, SI, hematocrit, hemoglobin, PaCo2, FiO2, base excess and mean airway pressure many of which are clinically relevant in hemodynamic monitoring. When using only ABP waveforms, approximate entropy of systolic, diastolic, shock index, CO (Zander), dicrotic notch amplitude, length of ABP pulse cycle and minimum of MAP, area under systole may also be used. When using only ECG waveforms, median of LF, ULF power; minimum of LF, VLF power; approximate entropy (which captures the regularity of the signal of heart rate), and Porta index may also be used. The LF component has been generally accepted as an indirect measure of the activity of both sympathetic and vagal systems and the heart rate is observed to be influenced by both divisions. Significant differences in frequency domain HRV metrics, specifically VLF, LF and HF, between stable and unstable patients during hemodialysis induced hypotension.

FIG. 4 illustrates a computer system for waveform-based hemodynamic instability warning, in accordance with a representative embodiment.

FIG. 4 illustrates a computer system, on which a method for waveform-based hemodynamic instability warning is implemented, in accordance with another representative embodiment.

The computer system 400 of FIG. 4 shows a complete set of components for a communications device or a computer device. However, a "controller" as described herein may be implemented with less than the set of components of FIG. 4, such as by a memory and processor combination. The computer system 400 may include some or all elements of one or more component apparatuses in a system for waveform-based hemodynamic instability warning herein, although any such apparatus may not necessarily include one or more of the elements described for the computer system 400 and may include other elements not described.

Referring to FIG. 4, the computer system 400 includes a set of software instructions that can be executed to cause the computer system 400 to perform some or all functions of any of the computer-based functions disclosed herein. The computer system 400 may operate as a standalone device or may be connected, for example, using a network 401, to other computer systems or peripheral devices. In embodiments, a computer system 400 performs logical processing based on digital signals received via an analog-to-digital converter.

In a networked deployment, the computer system 400 operates in the capacity of a server or as a client user computer in a server-client user network environment, or as a peer computer system in a peer-to-peer (or distributed) network environment. The computer system 400 can also be implemented as or incorporated into various devices, such as the workstation 140, the artificial intelligence controller 180 and/or the artificial intelligence training system 190 in FIG. 1, a stationary computer, a mobile computer, a personal computer (PC), a laptop computer, a tablet computer, or any other machine capable of executing a set of software instructions (sequential or otherwise) that specify actions to be taken by that machine. The computer system 400 can be incorporated as or in a device that in turn is in an integrated system that includes additional devices. In an embodiment, the computer system 400 can be implemented using electronic devices that provide voice, video or data communication. Further, while the computer system 400 is illustrated in the singular, the term "system" shall also be taken to include any collection of systems or sub-systems that individually or jointly execute a set, or multiple sets, of software instructions to perform one or more computer functions.

As illustrated in FIG. 4, the computer system 400 includes a processor 410. The processor 410 may be considered a representative example of the processor 152 of the controller 150 in FIG. 1, the processor 182 of the artificial intelligence controller 180 in FIG. 1 and/or the processor 192 of the artificial intelligence training system 190 in FIG. 1. The processor 410 executes instructions to implement some or all aspects of methods and processes described herein. The processor 410 is tangible and non-transitory. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The processor 410 is an article of manufacture and/or a machine component. The processor 410 is configured to execute software instructions to perform functions as described in the various embodiments herein. The processor 410 may be a general-purpose processor or may be part of an application specific integrated circuit (ASIC). The processor 410 may also be a microprocessor, a microcomputer, a processor chip, a controller, a microcontroller, a digital signal processor (DSP), a state machine, or a programmable logic device. The processor 410 may also be a logical circuit, including a programmable gate array (PGA), such as a field programmable gate array (FPGA), or another type of circuit that includes discrete gate and/or transistor logic. The processor 410 may be a central processing unit (CPU), a graphics processing unit (GPU), or both. Additionally, any processor described herein may include multiple processors, parallel processors, or both. Multiple processors may be included in, or coupled to, a single device or multiple devices.

The term "processor" as used herein encompasses an electronic component able to execute a program or machine executable instruction. References to a computing device comprising "a processor" should be interpreted to include more than one processor or processing core, as in a multi-core processor. A processor may also refer to a collection of processors within a single computer system or distributed among multiple computer systems. The term computing device should also be interpreted to include a collection or network of computing devices each including a processor or processors. Programs have software instructions performed by one or multiple processors that may be within the same computing device or which may be distributed across multiple computing devices.

The computer system 400 further includes a main memory 420 and a static memory 430, where memories in the computer system 400 communicate with each other and the processor 410 via a bus 408. Either or both of the main memory 420 and the static memory 430 may be considered representative examples of the memory 151 of the controller 150 in FIG. 1, the memory 181 of the artificial intelligence controller 180 in FIG. 1 and/or the memory 191 of the artificial intelligence training system 190 in FIG. 1. The main memory 420 and the static memory 430 may store instructions used to implement some or all aspects of methods and processes described herein. Memories described herein are tangible non-transitory computer readable storage mediums for storing data and executable software instructions and are non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The main memory 420 and the static memory 430 are articles of manufacture and/or machine components. The main memory 420 and the static memory 430 are computer-readable mediums from which data and executable software instructions can be read by a computer (e.g., the processor 410). Each of the main memory 420 and the static memory 430 may be implemented as one or more of random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM), digital versatile disk (DVD), floppy disk, blu-ray disk, or any other form of storage medium known in the art. The memories may be volatile or non-volatile, secure and/or encrypted, unsecure and/or unencrypted.

"Memory" is an example of a computer-readable storage medium. Computer memory is any memory which is directly accessible to a processor. Examples of computer memory include, but are not limited to RAM memory, registers, and register files. References to "computer memory" or "memory" should be interpreted as possibly being multiple memories. The memory may for instance be multiple memories within the same computer system. The memory may also be multiple memories distributed amongst multiple computer systems or computing devices.

As shown, the computer system 400 further includes a video display unit 450, such as a liquid crystal display (LCD), an organic light emitting diode (OLED), a flat panel display, a solid-state display, or a cathode ray tube (CRT), for example. Additionally, the computer system 400 includes an input device 460, such as a keyboard/virtual keyboard or touch-sensitive input screen or speech input with speech recognition, and a cursor control device 470, such as a mouse or touch-sensitive input screen or pad. The computer system 400 also optionally includes a disk drive unit 480, a signal generation device 490, such as a speaker or remote control, and/or a network interface device 440.

In an embodiment, as depicted in FIG. 4, the disk drive unit 480 includes a computer-readable medium 482 in which one or more sets of software instructions 484 (software) are embedded. The sets of software instructions 484 are read from the computer-readable medium 482 to be executed by the processor 410. Further, the software instructions 484, when executed by the processor 410, perform one or more steps of the methods and processes as described herein. In an embodiment, the software instructions 484 reside all or in part within the main memory 420, the static memory 430 and/or the processor 410 during execution by the computer system 400. Further, the computer-readable medium 482 may include software instructions 484 or receive and execute software instructions 484 responsive to a propagated signal, so that a device connected to a network 401 communicates voice, video or data over the network 401. The software instructions 484 may be transmitted or received over the network 401 via the network interface device 440.

In an embodiment, dedicated hardware implementations, such as application-specific integrated circuits (ASICs), field programmable gate arrays (FPGAs), programmable logic arrays and other hardware components, are constructed to implement one or more of the methods described herein. One or more embodiments described herein may implement functions using two or more specific interconnected hardware modules or devices with related control and data signals that can be communicated between and through the modules. Accordingly, the present disclosure encompasses software, firmware, and hardware implementations. Nothing in the present application should be interpreted as being implemented or implementable solely with software and not hardware such as a tangible non-transitory processor and/or memory.

In accordance with various embodiments of the present disclosure, the methods described herein may be implemented using a hardware computer system that executes software programs. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

From experiments described herein, utility of adding the ABP waveforms and ECG waveforms to clinical measurements and nurse-charted vital signs achieved an AUROC of 0.93, and using ABP waveforms and ECG waveforms in isolation achieved an AUROC of .85. Accordingly, waveform-based hemodynamic instability warning helps ensure adequacy of tissue perfusion and early detection of inadequacy of perfusion and end organ dysfunction. ECG waveforms and/or ABP waveforms can be used alone or together, along with laboratory tests and nurse-charted vital signs to predict patients with hemodynamic instability. The end-to-end waveform processing pipelines described herein may include beat segmentation, signal quality assessment and feature extraction, and lead to usable predictions of hemodynamic instability. Predicting patients who are likely to be hemodynamically unstable will allow for early intervention and treatment, which generally leads to better patient outcomes. Nevertheless, waveform-based hemodynamic instability warning is not limited as an application to particular details described herein, and instead is applicable to other embodiments for which alternative details may be feasible.

Although waveform-based hemodynamic instability warning has been described with reference to several exemplary embodiments, it is understood that the words that have been used are words of description and illustration, rather than words of limitation. Changes may be made within the purview of the appended claims, as presently stated and as amended, without departing from the scope and spirit of waveform-based hemodynamic instability warning in its aspects. Although waveform-based hemodynamic instability warning has been described with reference to particular means, materials and embodiments, waveform-based hemodynamic instability warning is not intended to be limited to the particulars disclosed; rather waveform-based hemodynamic instability warning extends to all functionally equivalent structures, methods, and uses such as are within the scope of the appended claims.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the FIG. s are to be regarded as illustrative rather than restrictive.

## Claims

1. An apparatus, comprising:

   a first interface (153) that interfaces at least one electrocardiogram monitor (155) monitoring a patient;
   a memory (151) that stores instructions, and
   a processor (152) that executes the instructions, wherein, when executed by the processor (152), the instructions cause the apparatus to:

   identify a plurality of heart beats from electrocardiogram waves received via the first interface (153) from the at least one electrocardiogram monitor (155);
   separate the plurality of heart beats into first temporal windows;
   extract features of the heart beats in each of the first temporal windows as first extracted features for each first temporal window;
   generate, based on the first extracted features, generated features across a second temporal window that includes a plurality of the first temporal windows;
   apply trained artificial intelligence to the generated features;
   predict hemodynamic instability for the patient based on applying the trained artificial intelligence to the generated features, and
   output an alert warning of the hemodynamic instability based on predicting the hemodynamic instability.

2. The apparatus of claim 1, further comprising:

   a second interface (154) that interfaces an arterial blood pressure monitor (155) monitoring the patient, wherein, when executed by the processor (152), the instructions further cause the apparatus to: segment arterial blood pressure waves received via the second interface (154) from the arterial blood pressure monitor (155) into individual pulse cycles, wherein the first temporal windows each include a plurality of the individual pulse cycles; and
   extract features of the arterial blood pressure waves in each of the first temporal windows as second extracted features for each first temporal window, wherein the generated features across the second temporal window are based additionally on the second extracted features,.

3. The apparatus of claim 1,
   wherein the alert includes a projection in advance of when the patient will enter a phase of hemodynamic instability based on an estimated likelihood passing a predetermined threshold.

4. The apparatus of claim 1,
   wherein, when executed by the processor (152), the instructions further cause the apparatus to:
   continuously identify the plurality of heart beats from the electrocardiogram waves for a plurality of the first temporal windows in the second temporal window.

5. The apparatus of claim 1, further comprising:

   a display that displays the alert,
   wherein the apparatus comprises a monitor (155).

6. A computer-implemented method, comprising:

   receiving, via a first interface (153) that interfaces at least one electrocardiogram monitor (155) monitoring a patient, a plurality of electrocardiogram waves;
   identifying (880) a plurality of heart beats from the electrocardiogram waves;
   separating the plurality of heart beats into first temporal windows;
   extracting features of the heart beats in each of the first temporal windows as first extracted features for each first temporal window;
   generating, based on the first extracted features, generated features across a second temporal window that includes a plurality of the first temporal windows;
   applying trained artificial intelligence to the generated features;
   predicting hemodynamic instability for the patient based on applying the trained artificial intelligence to the generated features, and
   outputting an alert warning of the hemodynamic instability based on predicting the hemodynamic instability.

7. The method of claim 6, further comprising:

   receiving, via a second interface (154) that interfaces an arterial blood pressure monitor (155) monitoring the patient, arterial blood pressure waves;
   segmenting the arterial blood pressure waves into individual pulse cycles, wherein the first temporal windows each include a plurality of the individual pulse cycles; and
   extracting features of the arterial blood pressure waves in each of the first temporal windows as second extracted features for each first temporal window, wherein the generated features across the second temporal window are based additionally on the second extracted features.

8. The method of claim 6, further comprising:

   labelling each of the plurality of heart beats with one of a plurality of predetermined labels based on characteristics of each of the plurality of heart beats; and
   excluding at least one heartbeat of the plurality of heart beats from application of the trained artificial intelligence based on applying a noise filter to the plurality of heart beats.

9. The method of claim 7, further comprising:

   identifying (880) at least one individual pulse cycle as abnormal; and
   excluding the at least one individual pulse cycle identified as abnormal from application of the trained artificial intelligence based on identifying (880) the at least one individual pulse cycle as abnormal.

10. The method of claim 6, wherein extracting features of the heart beats in each temporal window comprises:
    extracting heart rate variability as a first extracted feature for each temporal window.

11. A tangible non-transitory computer readable storage medium that stores a computer program, the computer program, when executed by a processor (152), causing a system (100) that includes the tangible non-transitory computer readable storage medium to:

identify a plurality of heart beats from electrocardiogram waves received via a first interface (153) from at least one electrocardiogram monitor (155);

separate the plurality of heart beats into first temporal windows;

extract features of the heart beats in each of the first temporal windows as first extracted features for each first temporal window;

generate, based on the first extracted features, generated features across a second temporal window that includes a plurality of the first temporal windows;

apply trained artificial intelligence to the generated features;

predict hemodynamic instability for a patient based on applying the trained artificial intelligence to the generated features, and

output an alert warning of the hemodynamic instability based on predicting the hemodynamic instability.

12. The tangible non-transitory computer readable storage medium of claim 11, wherein, when executed by the processor (152), the computer program further causes the system (100) that includes the tangible non-transitory computer readable storage medium to:

segment arterial blood pressure waves received via a second interface (154) from an arterial blood pressure monitor (155) into individual pulse cycles, wherein the first temporal windows each include a plurality of the individual pulse cycles; and

extract features of the arterial blood pressure waves in each of the first temporal windows as second extracted features for each first temporal window, wherein the generated features across the second temporal window are based additionally on the second extracted features.

13. The tangible non-transitory computer readable storage medium of claim 11, wherein, when executed by the processor (152), the computer program further causes the system (100) that includes the tangible non-transitory computer readable storage medium to:

label each of the plurality of heart beats with one of a plurality of predetermined labels based on characteristics of each of the plurality of heart beats; and

exclude at least one heartbeat of the plurality of heart beats from application of the trained artificial intelligence based on applying a noise filter to the plurality of heart beats.

14. The tangible non-transitory computer readable storage medium of claim 11, wherein, when executed by the processor (152), the computer program further causes the system (100) that includes the tangible non-transitory computer readable storage medium to:

identify at least one individual pulse cycle as abnormal; and

exclude the at least one individual pulse cycle identified as abnormal from application of the trained artificial intelligence based on identifying (880) the at least one individual pulse cycle as abnormal.

15. The tangible non-transitory computer readable storage medium of claim 11, wherein the tangible non-transitory computer readable storage medium is provided as a component of a monitor (155) that displays the alert.

**Patentansprüche**

1. Einrichtung, umfassend:

eine erste Schnittstelle (153), die mindestens einen Elektrokardiogrammmonitor (155), der einen Patienten überwacht, anbindet;

einen Speicher (151), der Anweisungen speichert, und

einen Prozessor (152), der die Anweisungen ausführt, wobei die Anweisungen, wenn sie von dem Prozessor (152) ausgeführt werden, bewirken, dass die Einrichtung:

aus Elektrokardiogrammwellen, die über die erste Schnittstelle (153) von dem mindestens einen Elektrokardiogrammmonitor (155) empfangen werden, eine Vielzahl von Herzschlägen identifiziert;

die Vielzahl von Herzschlägen in erste Zeitfenster aufteilt;

Merkmale der Herzschläge in jedem der ersten Zeitfenster als erste extrahierte Merkmale für jedes erste Zeitfenster extrahiert;

auf Basis der ersten extrahierten Merkmale generierte Merkmale über ein zweites Zeitfenster, das eine Vielzahl der ersten Zeitfenster beinhaltet, generiert;

trainierte künstliche Intelligenz auf die generierten Merkmale anwendet;

auf Basis der Anwendung der trainierten künstlichen Intelligenz auf die generierten Merkmale hämodynamische Instabilität für den Patienten vorhersagt, und

auf Basis der Vorhersage der hämodynamischen Instabilität einen Warnalarm bezüglich der hämodynamischen Instabilität ausgibt.

2.  Einrichtung nach Anspruch 1, weiter umfassend:

eine zweite Schnittstelle (154), die einen arteriellen Blutdruckmonitor (155), der den Patienten überwacht, anbindet, wobei die Anweisungen, wenn sie von dem Prozessor (152) ausgeführt werden, weiter bewirken, dass die Einrichtung:

arterielle Blutdruckwellen, die über die zweite Schnittstelle (154) von dem arteriellen Blutdruckmonitor (155) empfangen werden, in einzelne Pulszyklen segmentiert, wobei die ersten Zeitfenster jeweils eine Vielzahl der einzelnen Pulszyklen beinhalten; und

Merkmale der arteriellen Blutdruckwellen in jedem der ersten Zeitfenster als zweite extrahierte Merkmale für jedes erste Zeitfenster extrahiert, wobei die generierten Merkmale über das zweite Zeitfenster zusätzlich auf den zweiten extrahierten Merkmalen basieren.

3.  Einrichtung nach Anspruch 1,
wobei der Alarm eine Vorausprojektion, auf Basis einer geschätzten Wahrscheinlichkeit des Überschreitens einer vorbestimmten Schwelle beinhaltet, wann der Patient in eine Phase hämodynamischer Instabilität eintreten wird.

4.  Einrichtung nach Anspruch 1,
wobei die Anweisungen, wenn sie von dem Prozessor (152) ausgeführt werden, weiter bewirken, dass die Einrichtung:
kontinuierlich aus den Elektrokardiogrammwellen die Vielzahl von Herzschlägen für eine Vielzahl der ersten Zeitfenster im zweiten Zeitfenster identifiziert.

5.  Einrichtung nach Anspruch 1, weiter umfassend:

eine Anzeige, die den Alarm anzeigt,
wobei die Einrichtung einen Monitor (155) umfasst.

6.  Computerimplementiertes Verfahren, umfassend:

Empfangen einer Vielzahl von Elektrokardiogrammwellen über eine erste Schnittstelle (153), die mindestens einen Elektrokardiogrammmonitor (155), der einen Patienten überwacht, anbindet;

Identifizieren (880) einer Vielzahl von Herzschlägen aus den Elektrokardiogrammwellen;

Aufteilen der Vielzahl von Herzschlägen in erste Zeitfenster;

Extrahieren von Merkmalen der Herzschläge in jedem der ersten Zeitfenster als erste extrahierte Merkmale für jedes erste Zeitfenster;

Generieren, auf Basis der ersten extrahierten Merkmale, von generierten Merkmalen über ein zweites Zeitfenster, das eine Vielzahl der ersten Zeitfenster beinhaltet;

Anwenden von trainierter künstlicher Intelligenz auf die generierten Merkmale;

Vorhersagen von hämodynamischer Instabilität für den Patienten auf Basis der Anwendung der trainierten künstlichen Intelligenz auf die generierten Merkmale, und

Ausgeben eines Warnalarms bezüglich der hämodynamischen Instabilität auf Basis der Vorhersage der hämodynamischen Instabilität.

7.  Verfahren nach Anspruch 6, weiter umfassend:

Empfangen von arteriellen Blutdruckwellen über eine zweite Schnittstelle (154), die einen arteriellen Blutdruckmonitor (155), der den Patienten überwacht, anbindet;

Segmentieren der arteriellen Blutdruckwellen in einzelne Pulszyklen, wobei die ersten Zeitfenster jeweils eine Vielzahl der einzelnen Pulszyklen beinhalten; und

Extrahieren von Merkmalen der arteriellen Blutdruckwellen in jedem der ersten Zeitfenster als zweite extrahierte Merkmale für jedes erste Zeitfenster, wobei die generierten Merkmale über das zweite Zeitfenster zusätzlich auf den zweiten extrahierten Merkmalen basieren.

8. Verfahren nach Anspruch 6, weiter umfassend:

Markieren jedes der Vielzahl von Herzschlägen auf Basis von Kennzeichen jedes der Vielzahl von Herzschlägen mit einer aus einer Vielzahl von vorbestimmten Markierungen; und

Ausschließen, auf Basis der Anwendung eines Rauschfilters auf die Vielzahl von Herzschlägen, mindestens eines Herzschlags der Vielzahl von Herzschlägen aus der Anwendung der trainierten künstlichen Intelligenz.

9. Verfahren nach Anspruch 7, weiter umfassend:

Identifizieren (880) mindestens eines einzelnen Pulszyklus als abnormal; und

auf Basis des Identifizierens (880) des mindestens einen einzelnen Pulszyklus als abnormal, Ausschließen des mindestens einen einzelnen Pulszyklus, der als abnormal identifiziert wurde, aus der Anwendung der trainierten künstlichen Intelligenz.

10. Verfahren nach Anspruch 6, wobei das Extrahieren von Merkmalen der Herzschläge in jedem Zeitfenster umfasst:
Extrahieren der Herzfrequenzvariabilität als erstes extrahiertes Merkmal für jedes Zeitfenster.

11. Materielles nichtflüchtiges computerlesbares Speichermedium, das ein Computerprogramm speichert, wobei das Computerprogramm, wenn es von einem Prozessor (152) ausgeführt wird, bewirkt, dass ein System (100), das das materielle nichtflüchtige computerlesbare Speichermedium beinhaltet:

aus Elektrokardiogrammwellen, die über eine erste Schnittstelle (153) von mindestens einem Elektrokardiogrammmonitor (155) empfangen werden, eine Vielzahl von Herzschlägen identifiziert;

die Vielzahl von Herzschlägen in erste Zeitfenster aufteilt;

Merkmale der Herzschläge in jedem der ersten Zeitfenster als erste extrahierte Merkmale für jedes erste Zeitfenster extrahiert;

auf Basis der ersten extrahierten Merkmale generierte Merkmale über ein zweites Zeitfenster, das eine Vielzahl der ersten Zeitfenster beinhaltet, generiert;

trainierte künstliche Intelligenz auf die generierten Merkmale anwendet;

auf Basis der Anwendung der trainierten künstlichen Intelligenz auf die generierten Merkmale hämodynamische Instabilität für einen Patienten vorhersagt, und

auf Basis der Vorhersage der hämodynamischen Instabilität einen Warnalarm bezüglich der hämodynamischen Instabilität ausgibt.

12. Materielles nichtflüchtiges computerlesbares Speichermedium nach Anspruch 11, wobei das Computerprogramm, wenn es von dem Prozessor (152) ausgeführt wird, weiter bewirkt, dass das System (100), das das materielle nichtflüchtige computerlesbare Speichermedium beinhaltet:

arterielle Blutdruckwellen, die über eine zweite Schnittstelle (154) von einem arteriellen Blutdruckmonitor (155) empfangen werden, in einzelne Pulszyklen segmentiert, wobei die ersten Zeitfenster jeweils eine Vielzahl der einzelnen Pulszyklen beinhalten; und

Merkmale der arteriellen Blutdruckwellen in jedem der ersten Zeitfenster als zweite extrahierte Merkmale für jedes erste Zeitfenster extrahiert, wobei die generierten Merkmale über das zweite Zeitfenster zusätzlich auf den zweiten extrahierten Merkmalen basieren.

13. Materielles nichtflüchtiges computerlesbares Speichermedium nach Anspruch 11, wobei das Computerprogramm, wenn es von dem Prozessor (152) ausgeführt wird, weiter bewirkt, dass das System (100), das das materielle nichtflüchtige computerlesbare Speichermedium beinhaltet:

jeden der Vielzahl von Herzschlägen auf Basis von Kennzeichen jedes der Vielzahl von Herzschlägen mit einer aus einer Vielzahl von vorbestimmten Markierungen markiert; und

auf Basis der Anwendung eines Rauschfilters auf die Vielzahl von Herzschlägen mindestens einen Herzschlag der Vielzahl von Herzschlägen aus der Anwendung der trainierten künstlichen Intelligenz ausschließt.

14. Materielles nichtflüchtiges computerlesbares Speichermedium nach Anspruch 11, wobei das Computerprogramm, wenn es von dem Prozessor (152) ausgeführt wird, weiter bewirkt, dass das System (100), das das materielle nichtflüchtige computerlesbare Speichermedium beinhaltet:

mindestens einen einzelnen Pulszyklus als abnormal identifiziert; und

auf Basis des Identifizierens (880) des mindestens einen einzelnen Pulszyklus als abnormal den mindestens einen einzelnen Pulszyklus, der als abnormal identifiziert wurde, aus der Anwendung der trainierten künstlichen Intelligenz ausschließt.

15. Materielles nichtflüchtiges computerlesbares Speichermedium nach Anspruch 11,

wobei das materielle nichtflüchtige computerlesbare Speichermedium als Komponente eines Monitors (155), der den Alarm anzeigt, bereitgestellt wird.

**Revendications**

1. Appareil, comprenant :

une première interface (153) qui assure l'interface avec au moins un moniteur d'électrocardiogramme (155) surveillant un patient ;

une mémoire (151) qui stocke des instructions, et

un processeur (152) qui exécute les instructions, dans lequel, lorsqu'elles sont exécutées par le processeur (152), les instructions amènent l'appareil à :

identifier une pluralité de battements cardiaques à partir d'ondes d'électrocardiogramme reçues via la première interface (153) à partir de l'au moins un moniteur d'électrocardiogramme (155) ;

séparer la pluralité des battements cardiaques en premières fenêtres temporelles ;

extraire des caractéristiques des battements cardiaques dans chacune des premières fenêtres temporelles en tant que premières caractéristiques extraites pour chaque première fenêtre temporelle ;

générer, sur la base des premières caractéristiques extraites, des caractéristiques générées à travers une seconde fenêtre temporelle qui inclut une pluralité des premières fenêtres temporelles ;

appliquer une intelligence artificielle entraînée aux caractéristiques générées ;

prédire une instabilité hémodynamique du patient en appliquant l'intelligence artificielle entraînée aux caractéristiques générées, et

délivrer en sortie un avertissement d'alerte de l'instabilité hémodynamique sur la base de la prédiction de l'instabilité hémodynamique.

2. Appareil selon la revendication 1, comprenant en outre :
une seconde interface (154) qui assure l'interface avec un moniteur de pression artérielle (155) surveillant le patient, dans lequel, lorsqu'elles sont exécutées par le processeur (152), les instructions amènent en outre l'appareil à :

segmenter des ondes de pression artérielle reçues via la seconde interface (154) à partir du moniteur de pression artérielle (155) en cycles d'impulsions individuels, dans lequel les premières fenêtres temporelles incluent chacune une pluralité de cycles d'impulsions individuels ; et

extraire des caractéristiques des ondes de pression artérielle dans chacune des premières fenêtres temporelles en tant que secondes caractéristiques extraites pour chaque première fenêtre temporelle, dans lequel les caractéristiques générées à travers la seconde fenêtre temporelle sont basées en outre sur les secondes caractéristiques extraites.

3. Appareil selon la revendication 1,
dans lequel l'alerte inclut une projection à l'avance du moment auquel le patient va entrer dans une phase d'instabilité hémodynamique sur la base d'une probabilité estimée passant un seuil prédéterminé.

4. Appareil selon la revendication 1,

dans lequel, lorsqu'elles sont exécutées par le processeur (152), les instructions amènent en outre l'appareil à : identifier en continu la pluralité de battements cardiaques à partir des ondes d'électrocardiogramme pour une pluralité des premières fenêtres temporelles dans la seconde fenêtre temporelle.

5. Appareil selon la revendication 1, comprenant en outre :

un écran qui affiche l'alerte,
dans lequel l'appareil comprend un moniteur (155).

6. Procédé mis en œuvre par ordinateur, comprenant :

la réception, via une première interface (153) qui assure l'interface avec au moins un moniteur d'électrocardiogramme (155) surveillant un patient, d'une pluralité d'ondes d'électrocardiogramme ;
l'identification (880) d'une pluralité de battements cardiaques à partir des ondes d'électrocardiogramme ;
la séparation de la pluralité des battements cardiaques en premières fenêtres temporelles ;
l'extraction des caractéristiques des battements cardiaques dans chacune des premières fenêtres temporelles en tant que premières caractéristiques extraites pour chaque première fenêtre temporelle ;
la génération, sur la base des premières caractéristiques extraites, des caractéristiques générées à travers une seconde fenêtre temporelle qui inclut une pluralité des premières fenêtres temporelles ;
l'application d'une intelligence artificielle entraînée aux caractéristiques générées ;
la prédiction d'une instabilité hémodynamique du patient en appliquant l'intelligence artificielle entraînée aux caractéristiques générées, et
la délivrance en sortie d'un avertissement d'alerte de l'instabilité hémodynamique sur la base de la prédiction de l'instabilité hémodynamique.

7. Procédé selon la revendication 6, comprenant en outre :

la réception, via une seconde interface (154) qui assure l'interface avec un moniteur de pression artérielle (155) surveillant le patient, d'ondes de pression artérielle ;
la segmentation des ondes de pression artérielle en cycles d'impulsions individuels, dans lequel les premières fenêtres temporelles incluent chacune une pluralité des cycles d'impulsions individuels ; et
l'extraction des caractéristiques des ondes de pression artérielle dans chacune des premières fenêtres temporelles en tant que secondes caractéristiques extraites pour chaque première fenêtre temporelle, dans lequel les caractéristiques générées à travers la seconde fenêtre temporelle sont basées en outre sur les secondes caractéristiques extraites.

8. Procédé selon la revendication 6, comprenant en outre :

l'étiquetage de chacun de la pluralité de battements cardiaques avec une certaine d'une pluralité d'étiquettes prédéterminées sur la base de caractéristiques de chacun de la pluralité de battements cardiaques ; et
l'exclusion d'au moins un battement cardiaque de la pluralité de battements cardiaques d'une application de l'intelligence artificielle entraînée sur la base d'une application d'un filtre de bruit à la pluralité de battements cardiaques.

9. Procédé selon la revendication 7, comprenant en outre :

l'identification (880) d'au moins un cycle d'impulsions individuel comme anormal ; et
l'exclusion d'au moins un cycle d'impulsions individuel identifié comme anormal d'une application de l'intelligence artificielle entraînée sur la base d'une identification (880) de l'au moins un cycle d'impulsions individuel comme anormal.

10. Procédé selon la revendication 6, dans lequel l'extraction de caractéristiques des battements cardiaques dans chaque fenêtre temporelle comprend :
l'extraction d'une variabilité de fréquence cardiaque en tant que première caractéristique extraite pour chaque fenêtre temporelle.

11. Support de stockage tangible non transitoire lisible par ordinateur qui stocke un programme informatique, le programme informatique, lorsqu'il est exécuté par un processeur (152), amenant un système (100) qui inclut le support de stockage tangible non transitoire lisible par ordinateur à :

identifier une pluralité de battements cardiaques à partir d'ondes d'électrocardiogramme reçues via une première interface (153) à partir de l'au moins un moniteur d'électrocardiogramme (155) ;

séparer la pluralité des battements cardiaques en premières fenêtres temporelles ;

extraire des caractéristiques des battements cardiaques dans chacune des premières fenêtres temporelles en tant que premières caractéristiques extraites pour chaque première fenêtre temporelle ;

générer, sur la base des premières caractéristiques extraites, des caractéristiques générées à travers une seconde fenêtre temporelle qui inclut une pluralité des premières fenêtres temporelles ;

appliquer une intelligence artificielle entraînée aux caractéristiques générées ;

prédire une instabilité hémodynamique d'un patient en appliquant l'intelligence artificielle entraînée aux caractéristiques générées, et

délivrer en sortie un avertissement d'alerte de l'instabilité hémodynamique sur la base de la prédiction de l'instabilité hémodynamique.

12. Support de stockage tangible non transitoire lisible par ordinateur selon la revendication 11, dans lequel, lorsqu'il est exécuté par le processeur (152), le programme informatique amène en outre le système (100) qui inclut le support de stockage tangible non transitoire lisible par ordinateur à :

segmenter des ondes de pression artérielle reçues via une seconde interface (154) à partir du moniteur de pression artérielle (155) en cycles d'impulsions individuels, dans lequel les premières fenêtres temporelles incluent chacune une pluralité de cycles d'impulsions individuels ; et

extraire des caractéristiques des ondes de pression artérielle dans chacune des premières fenêtres temporelles en tant que secondes caractéristiques extraites pour chaque première fenêtre temporelle, dans lequel les caractéristiques générées à travers la seconde fenêtre temporelle sont basées en outre sur les secondes caractéristiques extraites.

13. Support de stockage tangible non transitoire lisible par ordinateur selon la revendication 11, dans lequel, lorsqu'il est exécuté par le processeur (152), le programme informatique amène en outre le système (100) qui inclut le support de stockage tangible non transitoire lisible par ordinateur à :

étiqueter chacun de la pluralité de battements cardiaques avec une certaine d'une pluralité d'étiquettes prédéterminées sur la base de caractéristiques de chacun de la pluralité de battements cardiaques ; et

exclure au moins un battement cardiaque de la pluralité de battements cardiaques d'une application de l'intelligence artificielle entraînée sur la base d'une application d'un filtre de bruit à la pluralité de battements cardiaques.

14. Support de stockage tangible non transitoire lisible par ordinateur selon la revendication 11, dans lequel, lorsqu'il est exécuté par le processeur (152), le programme informatique amène en outre le système (100) qui inclut le support de stockage tangible non transitoire lisible par ordinateur à :

identifier au moins un cycle d'impulsions individuel comme anormal ; et

exclure le au moins un cycle d'impulsions individuel identifié comme anormal de l'application de l'intelligence artificielle entraînée sur la base de l'identification (880) de l'au moins un cycle d'impulsions individuel comme anormal.

15. Support de stockage tangible non transitoire lisible par ordinateur selon la revendication 11,
dans lequel le support de stockage tangible lisible par ordinateur non transitoire est fourni en tant que composant d'un moniteur (155) qui affiche l'alerte.

SYSTEM
100

156

TOUCH PANEL

DISPLAY

152

151

102

MEMORY | PROCESSOR

154

ABP MONITOR(S)

I/F

CONTROLLER

155

ECG MONITOR(S)

I/F

150

WORKSTATION

153

101

140

182

MEMORY | PROCESSOR

181

ARTIFICIAL INTELLIGENCE
CONTROLLER

180

191

MEMORY | PROCESSOR

192

ARTIFICIAL INTELLIGENCE
TRAINING SYSTEM

190

FIG.1

FIG.2

S305 — RECEIVE ELECTROCARDIOGRAM WAVES

RECEIVE ARTERIAL BLOOD PRESSURE WAVES — S310

S315 — IDENTIFY HEART BEATS FROM ELECTROCARDIOGRAM WAVES

S325 — SEPARATE HEART BEATS INTO FIRST TEMPORAL WINDOWS

SEGMENT ARTERIAL BLOOD PRESSURE WAVES INTO PULSE CYCLE — S330

S335 — EXCLUDE HEART BEAT IF NOISY

EXCLUDE PULSE CYCLE IF ABNORMAL — S340

S345 — EXTRACT FEATURES OF HEART BEATS IN FIRST TEMPORAL WINDOWS

EXTRACT FEATURES OF ARTERIAL BLOOD PRESSURE IN FIRST TEMPORAL WINDOWS — S350

GENERATE FEATURES ACROSS SECOND TEMPORAL WINDOW — S357

APPLY TRAINED ARTIFICIAL INTELLIGENCE TO EXTRACTED FEATURES — S360

PREDICT HEMODYNAMIC INSTABILITY — S370

NO ← ABOVE THRESHOLD ? → NO — S380

YES

OUTPUT ALERT — S390

FIG.3

<u>400</u>

**410** PROCESSOR
INSTRUCTIONS

**420** MAIN MEMORY
INSTRUCTIONS

**430** STATIC MEMORY
INSTRUCTIONS

**440** NETWORK INTERFACE DEVICE

**401** NETWORK

**408**

**450** VIDEO DISPLAY

**460** ALPHA-NUMERIC INPUT DEVICE

**470** CURSOR CONTROL DEVICE

**480** DRIVE UNIT
**482** COMPUTER READABLE MEDIUM
**484** INSTRUCTIONS

**490** SIGNAL GENERATION DEVICE

FIG.4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015200750 A1

**Non-patent literature cited in the description**

- **HANQING CAO et al.** Predicting ICU hemodynamic instability using continuous multi parameter trends. *ENGINEERING IN MEDICINE AND BIOLOGY SOCIETY, 2008. 30TH ANNUAL INTERNATIONAL CONFERENCE OF THE IEEE*, 20 August 2008